# EUROPEAN PATENT SPECIFICATION

(11) **EP 1 206 435 B1**
(45) Date of publication and mention of the grant of the patent: **17.11.2004**
(21) Application number: 00962037.8
(22) Date of filing: 02.08.2000
(51) Int. Cl.: C07C 51/42, C07C 59/215, C07C 59/08, C07C 59/265, C07C 59/105, C12P 7/60

(54) **PROCESS FOR THE RECOVERY OF ORGANIC ACIDS**
VERFAHREN ZUM GEWINNEN VON ORGANISCHEN SÄUREN
PROCEDE POUR LA RECUPERATION D'ACIDES ORGANIQUES

(30) Priority: 03.08.1999 US 147031 P
(43) Date of publication of application: 22.05.2002
(73) Proprietor: ARCHER-DANIELS-MIDLAND COMPANY, Decatur, Illinois 62526 (US)
(72) Inventor: MOORE, Kevin, M., Mt. Zion, IL 62549 (US); SANBORN, Alexandra, J., Lincoln, IL 62656 (US)
(74) Representative: Grünecker, Kinkeldey, Stockmair & Schwanhäusser Anwaltssozietät
(86) International application number: PCT/US2000/040538
(87) International publication number: WO 2001/009074

(56) References cited:
- EP-A- 0 174 624
- EP-A- 0 805 210

## Description

### Field of the Invention

The present invention relates to a process for the recovery and purification of organic acids from an aqueous solution, such as a fermentation broth.

### Related Art

Organic acids are important chemicals of commerce, having many uses in the food and pharmaceutical industries. Lactic acid, for example, can be added to a variety of foodstuffs as a preservative and is used in medical preparations and as a monomer in the manufacture of biodegradable plastics used in sutures, prosthetics and controlled release drug delivery systems.

Organic acids can be produced either by chemical synthesis or by microbial fermentation. Historically, organic acids were produced from animal fat or vegetable oil sources or from petroleum sources in substantially nonaqueous systems. However, since a number of microorganisms are known to produce valuable organic acids. methods have been developed for the recovery of organic acids produced by microbial fermentation. *See. e*.*g*., U.S. Patent Nos. 5,681,728; 5,034,105; 5,002,881; and 4,882,277.

In the process for preparing organic acids, the step of fermentation is relatively simple. However, the steps of recovery and purification of the product, *i.e.* an organic acid, are usually complicated with poor efficiency. For example, recovery of a representative organic acid, 2-keto-L-gulonic acid. is made as follows.

2-keto-L-gulonic acid is a significant intermediate in the preparation of L-ascorbic acid (vitamin C), an essential nutrient. In the past. 2-keto-L-gulonic acid has been synthesized on an industrial scale using the Reichstein method (*Helvetica Chimica Acta 17*:311 (1934)). This method, however, has a number of disadvantages for commercial application, including the use of large quantities of solvents and the involvement of a number of complex reaction steps.

Accordingly, as an alternative to the Reichstein method, a number of processes employing one or more microorganisms have been developed for the commercial production of 2-keto-L-gulonic acid by fermentation. U.S. Patent No. 2,421,611, for example, discloses a method involving microbial oxidation of D-glucose to 5-keto-D-gluconic acid, followed by chemical or microbial reduction to L-idonic acid and subsequent microbial oxidation to 2-keto-L-gulonic acid. Fermentative pathways involving oxidation of L-sorbose to 2-keto-L-gulonic acid via a sorbosone intermediate have also been developed using, for example, *Gluconobacter oxydans* (U.S. Patent Nos. 4,935,359; 4,960.695; and 5,312,741), *Pseudogluconobacter saccharokelogenes* (U.S. Patent No. 4,877,735), *Pseudomonas sorbosoxidans* (U.S. Patent Nos. 4,892,823 and 4,933,289), and mixtures of microorganisms (U.S. Patent Nos. 3,234,105; 3,907,639; and 3,912,592).

Similar to fermentation processes utilized for the manufacture of other organic acids, the 2-keto-L-gulonic acid which results as a metabolic product is usually neutralized by the addition of a base, *e*.*g*., sodium hydroxide or calcium hydroxide. in order to control the pH value and maintain favorable fermentation conditions. The product of the fermentation is an aqueous. biomass-containing fermentation solution in which the 2-keto-L-gulonic acid salt, e.g.. the sodium. potassium. ammonium or calcium salt, is present in dissolved form. However, the free organic acids and their derivatives are the articles of commercial interest.

For example, in the industrial manufacture of L-ascorbic acid, the fermentatively produced 2-keto-L-gulonic acid must be transferred into an organic solvent. such as a lower alcohol. The salt form of 2-keto-L-gulonic acid. however, is practically insoluble in organic solvents. Therefore, for producing L-ascorbic acid with an industrial advantage, it is most preferable to employ the synthetic intermediate 2-keto-L-gulonic acid as a free acid.

Prior to conversion into ascorbic acid, however, 2-keto-L-gulonic acid must first be isolated from the fermentation broth. As described in U.S. Patent No. 4,990,441, for example, 2-keto-L-gulonic acid can be recovered from a fermentation broth by a process comprising the steps of: (a) removing insoluble material from the broth by centrifugation in the presence of a flocculating agent, filtration in the presence of a flocculating agent and a filtration additive, or ultrafiltration; (b) removing inorganic cations by acidification; and (c) isolating 2-keto-L-gulonic acid by crystallization and drying. High yields of 2-keto-L-gulonic acid are difficult to obtain by this method, however, due to the number of steps required and the high solubility of 2-keto-L-gulonic acid in the crystallization mother liquor.

U.S. Patent No. 5,852,211 describes a process for the conversion of the sodium salt of 2-keto-L-gulonic acid, which is present in an aqueous fermentation solution, into an alcoholic solution of the free acid. The disclosed process comprises the steps of: (a) crystallizing sodium 2-keto-L-gulonate monohydrate from an aqueous fermentation solution; (b) separating the sodium 2-keto-L-gulonate monohydrate crystals from the aqueous fermentation solution; (c) suspending the sodium 2-keto-L-gulonate monohydrate in a lower alcohol with acid at a pH in a range from about 1.5 to about 3.5 whereby the acid is converted to the insoluble sodium salt of the acid and the sodium 2-keto-L-gulonate monohydrate is converted to free 2-keto-L-gulonic acid; and (d) removing the sodium salt of the acid to obtain an alcoholic solution of 2-keto-L-gulonic acid. Due to the number of steps and the high solubility of the sodium 2-keto-L-gulonic acid that remains in the crystallization mother liquor, however, high yields of 2-keto-L-gulonic acid are likewise difficult to achieve.

EP-A-0 1740624 relates to a process for the production of lactic acid ester and describes the fermentation of a calcium acetate solution containing lactic acid, filtration and spray drying of said solution, mixing of the crude calcium acetate containing lactic acid with isobutanol, toluene and concentrated hydrochloric acid, neutralising the organic phase with sodium hydroxide and a final distillation step. In EP-A-0 1740624 the fermentation broth is filtered while being hot prior to the reaction with alcohol. A yield of 89% of lactic acid butyl ester was obtained (see example 1) in EP-A-0 1740624.

EP-A-0 805 210 relates to a process for the conversion of the sodium salt of 2-keto-L-gulonic acid being present in a aqueous fermentation solution into an alcoholic solution of the free acid and, if desired, into an alkyl ester of the acid.

Known methods for the recovery and purification of other organic acids produced by fermentation, such as lactic or succinic acid, also contain numerous steps and/or produce less than optimal yields. See, e.g. U.S. Patent Nos. 5,681,728; 5,522,995; 5,503,750; and 5,034,105. Accordingly, there remains a need for a simplified process for the concentration, recovery and purification of organic acids from aqueous solutions, such as fermentation broths, in high yields.

### Summary of the Invention

It is therefore an object of the present invention to provide a simplified process for the recovery and purification of organic acids in high yields from a fermentation broth. Other objects, features and advantages of the present invention will be set forth in the detailed description of preferred embodiments that follows, and in part will be apparent from the description or can be learned by practice of the invention. These objects and advantages of the invention will be realized and attained by the methods particularly pointed out in the written description and claims hereof.

These and other objects are accomplished by the methods of the present invention, which, in a first embodiment, are broadly directed to a process for the recovery of an organic acid from a fermentation broth which comprises drying the fermentation broth to obtain a dried product, adding the dried fermentation product to a lower alcohol in the presence of an acid, and then removing the insoluble material to obtain the organic acid by filtration. Other preferred embodiments of the present invention will be described in more detail below.

It is to be understood that both the foregoing general description and the following detailed description are exemplary and explanatory only and are intended to provide further explanation of the invention as claimed.

### Detailed Description of the Preferred Embodiments

The objects discussed above are solved by the embodiments of the present invention. In one aspect of the present invention there is provided a process for the recovery of an organic acid from a fermentation broth comprising (a) drying said fermentation broth to obtain a dried product; (b) adding said dried product (a) to a lower alcohol, selected from the group consisting of methanol, ethanol, propanol, butanol or glycol, in the presence of an acid; and (c) removing insolubles to obtain an organic acid, wherein the process for removing insolubles comprises filtration.

Also a preferred embodiment is a process for the recovery of an organic acid from a fermentation broth comprising: (a) drying said fermentation broth to obtain a dried product;(b) adding said dried product (a) to a lower alcohol, selected from the group consisting of methanol, ethanol, propanol, butanol or glycol, to obtain an alcoholic suspension; (c) adding an acid to said alcoholic suspension (b); and (d) removing the insolubles to obtain an organic acid, wherein the process for removing insolubles comprises filtration.

Further, a preferred embodiment of this invention is a process for the recovery of an organic acid ester from a fermentation broth comprising: (a) drying said fermentation broth to obtain a dried product; (b) adding said dried product (a) to a lower alcohol, selected from the group consisting of methanol, ethanol, propanol, butanol or glycol, in the presence of an acid; (c) esterifying the free organic acid to the corresponding ester; and (d) removing insolubles to obtain an organic acid ester, wherein the process for removing insolubles comprises filtration.

Moreover, a preferred embodiment of this invention is a process for the recovery of an organic acid from a fermentation broth comprising: (a) drying said fermentation broth to obtain a dried product, wherein said drying occurs without prior removal of insolubles from said organic acid-containing fermentation broth; (b) adding said dried product (a) to a lower alcohol, selected from the group consisting of methanol, ethanol, propanol, butanol or glycol, in the presence of an acid; and removing insolubles to obtain an organic acid.

As used herein, the term "organic acid" or grammatical equivalents means a substituted or unsubstituted alkyl group containing one or more carboxyl groups, --COOH, *i*.*e*. a carboxyl acid. The organic acids can be obtained from a fermentation conversion of saccharides such as starch, sucrose or glucose or from n-paraffins.

A non-exclusive list of examples oforganic acids which can be recovered using the inventive process includes lactic acid, 2-keto-L-gulonic acid, tartaric acid, citric acid, acetic acid, maleic acid, malic acid, malonic acid, succinic acid, salicylic acid, glycolic acid, glutaric acid, gluconic acid, benzoic acid, formic acid, propionic acid, pivalic acid, oxalic acid, toluic acid, stearic acid, ascorbic acid, pamoic acid, glutamic acid. fumaric acid or mixtures thereof. In a preferred embodiment, the recovered organic acid is lactic acid, 2-keto-L-gulonic acid, citric acid or gluconic acid.

It has been surprisingly and advantageously found that organic acids of a high purity can be recovered at high yields using the whole dried fermentation broth as the starting material. With the aid of the process in accordance with the invention, organic acids such as lactic acid and 2-keto-L-gulonic acid. which usually occur as a dissolved salt in the aqueous fermentation solution, can be recovered in a relatively simple and economical manner into an alcoholic solution of the free acid.

Following recovery of the organic acids according to the present invention, the organic acids can be further treated, if necessary, in order to render them suitable for their intended purpose. For example, the thus-obtained solution of 2-keto-L-gulonic acid can subsequently be converted into ascorbic acid in any known manner. *See, e*.*g*., U.S. Patent No. 5,391,770.

Any fermentation broth containing the desired organic acid(s), a precursor thereof or its water-soluble salt can be used in the present invention. Such a fermentation broth, which is effected prior to the actual process in accordance with the invention, can be obtained by any conventional means, such as microbial fermentation. *See, e.g.,* U.S. Patent Nos. 5,834,231 and 5,705,373. Further, the microbes used in the fermentation process may be Protists (yeast or fungi) or bacteria, and the fermentation itself may be either aerobic or anaerobic.

The fermentation broth used in the present invention is typically, but not always, a fermentation broth produced by the cultivation of one or more microorganisms that produce a specific organic acid(s) and/or a precursor thereof. Any microorganism(s) which produces the desired organic acid can be used to prepare the fermentation broth. The organic acid producing microorganisms which can be used, for example, include bacteria belonging to the genera *Lactobacillus*, *Pseudogluconobacter*, *Pseudomonas, Corynebacterium Acetobacter*, *Gluconobacter*, *Aspergillus*, *Brevibacterium* and bacteria belonging to the genus *Erwinia*. *See, e*.*g*., Atkinson and Mavituna, *Biological Engineering and Biotechnology Handbook* 421 (1983).

A temperature suitable for each species of microorganism used is employed as the fermentation temperature. It is usually from about 25 °C to about 60°C. In addition, most of the organic acid-producing microorganisms have an acid sensitivity which require the medium to have a pH from about 3 to about 9.

The composition of the fermentation medium where an organic acid is produced using the above-mentioned microorganisms can be any one which is suitable for an organic acid producing microorganism to be used. In addition to water and an insoluble biomass, these fermentation broths generally contain the nutrients required by the microorganism(s) being employed to produce the organic acid. These nutrients include, but are not limited to, amino acids, inorganic and/or organic salts, carbohydrates, and various vitamins and growth factors.

The insolubles, *e.g.*, microorganisms which form the biomass and salts, are removed at the end of fermentation and can be removed by any known mechanical separation technique. Such techniques include, but are not limited to, filtration, *e*.*g*., ultrafiltration and microfiltration, and separation, *e*.*g*., centrifugation and decanting, by which, on the whole, only undissolved and/or relatively high molecular weight substances are removed. Most preferably, at least part of the insolubles are removed by ultrafiltration.

The fermentation broth can be concentrated prior to drying. In one embodiment, the process for concentrating the fermentation broth is by evaporation.

Therefore, in one embodiment of the invention, the whole fermentation broth can be used as the starting material for the recovery of the organic acid.

In the inventive process, the fermentation broth is dried to obtain a dried product. The fermentation broth can be dried by any known process, *e*.*g*., by means of a spray dryer, a spin flash dryer or a fluidized bed dryer. In a preferred embodiment, the fermentation broth is dried by the use of a spray dryer. The moisture percentage of the dried product is from about 0.1% to about 20% moisture, more preferably from about 0.1% to about 10%. The dried product which is obtained contains, among other substances, the desired organic acid and all or part or none of the biomass. It was surprising and particularly advantageous that the fermentation broth could be dried, *e*.*g*., spray dried, to obtain an easily handled free flowing powder.

According to the inventive process, the dried fermentation broth containing, *inter alia*, the desired organic acid, a precursor thereof or its salt, is added to a lower alcohol in the presence of an acid. In a preferred embodiment, the concentration of the organic acid added to the lower alcohol is from 50 g/L to 100 g/L. The lower alcohol employed in the inventive process include methanol, ethanol, propanol, butanol and glycol. The lower alcohol may be anhydrous methanol or ethanol.

In general, the amount of lower alcohol and acid employed in the inventive process can be any amount which allows for the selective recovery of the desired organic acid. In addition, the amount of acid added will be proportional to the amount of dried product. In a preferred embodiment, the acid is added in stoichiometric proportions or in excess, preferably 1.2 equivalents of acid is added.

The acid can be any acid which allows for the selective recovery of the desired organic acid. For example, a strong acid of low water content can be used in the inventive process. The water content of the acid is not critical for the process. However, the concentration of water in the resulting organic acid/alcohol solution determines the equilibrium conversion of a subsequent esterification. Therefore, from an industrial-economical point of view, acids of low water content, *i*.*e*., acids more appropriately denoted as "concentrated" are preferably used. The water content of the acid may be about 15% or less. Examples of such acids are sulphuric acid, nitric acid, hydrochloric acid, hydrobromic acid and phosphoric acid, and even gaseous hydrogen chloride. In a preferred embodiment. concentrated sulphuric acid or hydrochloric acid is used. More preferably, concentrated sulfuric acid is used.

In one embodiment of the invention, the dried fermentation product can first be suspended in the lower alcohol prior to the addition of the acid. The desired organic acid can also be obtained in another embodiment of the invention whereby the dried product is added together with the acid to a lower alcohol. It is therefore understood that both the simultaneous and subsequent addition of acid are included within the scope of the inventive process, although the subsequent addition of acid is preferred.

Preferably, the temperature at which the dehydration and acid reactions are carried out lies in the range from 25° C to 60° C. In addition, the resulting organic acid should be soluble in the reaction mixture.

In another embodiment of the invention, there is provided a process for the recovery of an organic acid ester from a fermentation broth. Carboxylic acids react with alcohols in the presence of a strong acid, such as sulfuric acid, to produce esters. Under certain conditions the obtained free organic acid of the present invention, together with a lower alcohol in the presence of an acid, can be converted into its corresponding organic acid lower alkyl ester. Accordingly, it is understood that the recovered free organic acid as well as the corresponding organic acid ester are included within the scope of the inventive process.

According to the inventive process, the insolubles are subsequently removed from the solution to obtain the desired organic acid. After the dried product is added to a lower alcohol in the presence of an acid, insoluble salts, *e*.*g*., Na₂SO₄, CaSO₄, K₂SO₄, (NH₄)₂SO₄, can be removed readily from the reaction mixture. The removal ofthe insoluble salts, as well as other insoluble substances, *e*.*g*., residues of biomass or proteins, can be carried out by any known process, such as filtration and/or centrifugation. In apreferred embodiment, the insolubles are removed by filtration. The resulting alcoholic filtrate containing the desired organic acid has a very high purity.

Therefore, in accordance with the recovery process of the present invention, organic acids of a high purity, *i*.*e*., greater than about 80%. can be recovered in high yields, *i*.*e*., greater than about 90%, preferably 95% to 99%, from the whole fermentation solution containing various impurities with fewer steps as compared with conventional methods. The disadvantages of prior art processes, such as the complete removal of biomass and proteins; the use of cation exchangers to remove metal ions from the aqueous fermentation solutions as well as the crystallization of the organic acid, are thereby avoided.

All patents and publications cited in this disclosure are indicative of the level of skill of those skilled in the art to which this invention pertains.

Having now generally described the invention, the same will be more readily understood through reference to the following Examples which are provided by way of illustration, and are not intended to be limiting of the present invention, unless specified.

### Example 1

A fermentation broth was prepared as described in U.S. Patent No. 5,834,231 containing 84 g/L 2-keto-L-gulonic acid, 4 g/L sorbose and 136 g/L total dry solids and spray-dried on a high pressure nozzle sprayer (APV Americas; Tonawanda, New York). The feed was preheated to 170 °F (76,67°C) and dried with a 495 °F (257,22°C) inlet and a 198 °F 92,22°C outlet temperature. A total of 8 gallons (30,28 liter) of feed was dried to give 10.2 lbs (4,63 kg) of dried product at 8 % moisture.

25 g of the spray dried product was slurried into 250 ml of anhydrous methanol. 3.2 ml of sulfuric acid was added to the solution over 20 minutes while stirring. The slurry was stirred for an additional 60 minutes, filtered and the solids washed with 150 ml of additional methanol. The product filtrate of 320 ml contained 35 g/L 2-keto-L-gulonic acid and 1.7 g/L of 2-keto-L-gulonic acid methyl ester. The cake of 12 grams contained 3.8 % 2-keto-L-gulonic acid and 0.4 % 2-keto-L-gulonic acid methyl ester, for a yield of 96 %.

### Example 2

A fermentation broth made similar to that in Example 1 was first ultrafiltered using a 100,000 molecular weight cutoff membrane to remove cell solids, and then evaporated to a 1.2 g/ml density before spray drying. The feed was preheated to 165 °F (73,89°C), and dried with a 495 °F (257,22°C) inlet and 200 °F (93,33°C) outlet temperature. Feed dry solids was 39.2 wt %. A total of 10 gallons (37,85 liter) of feed was dried, to give 42 lbs. (19,05 kg) of dry product at 15 % moisture.

36 g of the spray dried product was slurried into 250 ml of anhydrous methanol. 4.2 ml of concentrated sulfuric acid was added over 20 minutes. The slurry was stirred for an additional 60 minutes, filtered, and washed with 150 ml of methanol. The product filtrate of 335 ml contained 67 g/L 2-keto-L-gulonic acid and 1 g/L 2-keto-L-gulonic acid methyl ester. The cake of 31 g contained 3.0 % 2-keto-L-gulonic acid with no methyl ester, for a yield of 96 %.

### Example 3

10 ml of product filtrate obtained by the procedure of Example 2, but containing 71.9 g/L of 2-keto-L-gulonic acid, was placed in a test tube. To this solution was added 0.14 ml of concentrated sulfuric acid. The test tube was placed in an oven at 60 °C for 1 hour to give 1.5g/L of 2-keto-L-gulonic acid and 77.1 g/L of methyl ester.

### Example 4

A fermentation broth of calcium lactate. made from the fermentation of *Lactobacillus casei* on dextrose, was ultrafiltered and spray dried, using the procedures of Examples 1 and 2, to obtain a dry product containing 70.3 wt% lactic acid. 25 g of the spray dried calcium lactate was slurried into 500 ml anhydrous ethanol. 6.4 ml of concentrated sulfuric acid was added over 30 minutes. The slurry was stirred for an additional 60 minutes, filtered, and washed two times with 100 ml ethanol. The filtrate of 490 ml contained 59.1 g/L lactic acid and the wash of 195 ml contained 20.1 g/L lactic acid. The cake of 25 g contained 1.8g/kg lactic acid for a yield of 99%.

### Example 5

17.5 g of the spray dried calcium lactate was slurried into 125 ml of anhydrous ethanol. 4.9 ml of concentrated sulfuric acid was added over 30 minutes. The slurry was allowed to heat to 75 °C with stirring for one hour. The slurry was filtered and washed two times with 100 ml of ethanol. The filtrate of 200 ml contained 67.9 g/L ethyl lactate and 1.6 g/L lactic acid for a total yield of 86.6%.

## Claims

1. A process for the recovery of an organic acid from a fermentation broth comprising:
(a) drying said fermentation broth to obtain a dried product;
(b) adding said dried product (a) to a lower alcohol, selected from the group consisting of methanol, ethanol, propanol, butanol or glycol, in the presence of an acid; and
(c) removing insolubles to obtain an organic acid, wherein the process for removing insolubles comprises filtration.

2. A process for the recovery of an organic acid from a fermentation broth comprising:
(a) drying said fermentation broth to obtain a dried product;
(b) adding said dried product (a) to a lower alcohol, selected from the group consisting of methanol, ethanol, propanol, butanol or glycol, to obtain an alcoholic suspension;
(c) adding an acid to said alcoholic suspension (b); and
(d) removing the insolubles to obtain an organic acid, wherein the process for removing insolubles comprises filtration.

3. A process for the recovery of an organic acid ester from a fermentation broth comprising:
(a) drying said fermentation broth to obtain a dried product;
(b) adding said dried product (a) to a lower alcohol, selected from the group consisting of methanol, ethanol, propanol, butanol or glycol, in the presence of an acid;
(c) esterifying the free organic acid to the corresponding ester; and
(d) removing insolubles to obtain an organic acid ester, wherein the process for removing insolubles comprises filtration.

4. The process of claim 1,2 or 3, further comprising removing the insolubles in said fermentation broth prior to the drying of step (a).

5. The process of claim 4, wherein said insolubles are removed by filtration.

6. The process of claim 5, wherein said insolubles are removed by ultrafiltration.

7. The process of claim 1,2 or 3, wherein at step (b) the concentration of said organic acid added to said lower alcohol is from 50 g/L to 100 g/L.

8. The process of claim 1,2 or 3, wherein at step (a) the process for drying comprises spray drying said fermentation broth.

9. The process of claim 1, wherein the reaction temperature at step (b) is from 25°C to 60°C.

10. The process of claim 2 or 3, wherein the reaction temperature at steps (b) and (c) is from 25°C to 60°C.

11. The process of claim 1 or 3, wherein at step (b) 1.2 equivalents of acid is added.

12. The process of claim 2, wherein at step (c) 1.2 equivalents of acid is added.

13. The process of claim 1 or 3, wherein at step (b) said acid is selected from the group consisting of sulphuric acid, nitric acid, hydrobromic acid, hydrochloric acid and phosphoric acid.

14. The process of claim 2, wherein at step (c) said acid is selected from the group consisting of sulphuric acid, nitric acid, hydrobromic acid, hydrochloric acid and phosphoric acid.

15. The process of claim 13, wherein at step (b) said acid is sulphuric acid.

16. The process of claim 14, wherein at step (c) said acid is sulphuric acid.

17. The process of claim 1,2 or 3, wherein said organic acid comprises lactic acid, 2-keto-L-gulonic acid, citric acid or gluconic acid.

18. The process of claim 17, wherein said organic acid is 2-keto-L-gulonic acid.

19. The process of claim 1, further comprising esterifying said organic acid (c) to the corresponding ester.

20. The process of claim 2, further comprising esterifying said organic acid (d) to the corresponding ester.

21. A process for the recovery of an organic acid from a fermentation broth comprising:
(a) drying said fermentation broth to obtain a dried product, wherein said drying occurs without prior removal of insolubles from said organic acid-containing fermentation broth;
(b) adding said dried product (a) to a lower alcohol, selected from the group consisting of methanol, ethanol, propanol, butanol or glycol, in the presence of an acid; and
(c) removing insolubles to obtain an organic acid;

22. The process of claim 21, wherein at step (b) the concentration of said organic acid added to said lower alcohol is from 50 g/L to 100 g/L.

23. The process of claim 21, wherein at step (a) the process for drying comprises spray drying said fermentation broth.

24. The process of claim 21, wherein the reaction temperature at step (b) is from 25°C to 60°C.

25. The process of claim 21, wherein at step (b) 1.2 equivalents of acid is added.

26. The process of claim 21, wherein at step (b) said acid is selected from the group consisting of sulphuric acid, nitric acid, hydrobromic acid, hydrochloric acid and phosphoric acid.

27. The process of claim 26, wherein at step (b) said acid is sulphuric acid.

28. The process of claim 21, wherein said organic acid comprises lactic acid, 2-keto-L-gulonic acid, citric acid or gluconic acid.

29. The process of claim 28, wherein said organic acid is 2-keto-L-gulonic acid.

30. The process of claim 21, wherein at step (c) the process for removing insolubles comprises filtration.

31. The process of claim 21, further comprising esterifying said organic acid (c) to the corresponding ester.

## Patentansprüche

1. Verfahren zur Gewinnung einer organischen Säure aus einer Fermentationsbrühe, umfassend:
(a) Trocknen der Fermentationsbrühe, um ein getrocknetes Produkt zu erhalten;
(b) Zugeben des getrockneten Produkts (a) zu einem niedrigen Alkohol, gewählt aus der Gruppe bestehend aus Methanol, Ethanol, Propanol, Butanol oder Glykol, in Gegenwart einer Säure; und
(c) Abtrennen von Unlöslichem um eine organische Säure zu erhalten, wobei das Verfahren zum Abtrennen von Unlöslichem Filtration umfasst.

2. Verfahren zur Gewinnung einer organischen Säure aus einer Fermentationsbrühe, umfassend:
(a) Trocknen der Fermentationsbrühe, um ein getrocknetes Produkt zu erhalten;
(b) Zugeben des getrockneten Produkts (a) zu einem niedrigen Alkohol, gewählt aus der Gruppe bestehend aus Methanol, Ethanol, Propanol, Butanol oder Glykol, um eine alkoholische Suspension zu erhalten;
(c) Zugeben einer Säure zu der alkoholischen Suspension (b); und
(d) Abtrennen von Unlöslichem, um eine organische Säure zu erhalten, wobei das Verfahren zum Abtrennen von Unlöslichem Filtration umfasst.

3. Verfahren zur Gewinnung eines organischen Säureesters aus einer Fermentationsbrühe, umfassend:
(a) Trocknen der Fermentationsbrühe, um ein getrocknetes Produkt zu erhalten;
(b) Zugeben des getrockneten Produkts (a) zu einem niedrigen Alkohol, gewählt aus der Gruppe bestehend aus Methanol, Ethanol, Propanol, Butanol oder Glykol, in Gegenwart einer Säure;
(c) Verestern der freien organischen Säure zu dem korrespondierenden Ester; und
(d) Abtrennen von Unlöslichem, um einen organischen Säureester zu erhalten, wobei das Verfahren zum Abtrennen von Unlöslichem Filtration umfasst.

4. Verfahren nach Anspruch 1, 2 oder 3, welches außerdem das Abtrennen von Unlöslichem aus der Fermentationsbrühe vor dem Trocknungsschritt (a) umfasst.

5. Verfahren nach Anspruch 4, worin das Unlösliche durch Filtration abgetrennt wird.

6. Verfahren nach Anspruch 5, worin das Unlösliche durch Ultrafiltration abgetrennt wird.

7. Verfahren nach Anspruch 1, 2 oder 3, worin in Schritt (b) die Konzentration der zu dem niedrigem Alkohol gegebenen organischen Säure im Bereich von 50 g/l bis 100 g/l liegt.

8. Verfahren nach Anspruch 1, 2 oder 3, worin in Schritt (a) das Verfahren zum Trocknen Sprühtrocknen der Fermentationsbrühe umfasst.

9. Verfahren nach Anspruch 1, worin die Reaktionstemperatur in Schritt (b) 25° bis 60° C beträgt.

10. Verfahren nach Anspruch 2 oder 3, worin die Reaktionstemperatur in den Schritten (b) und (c) 25° bis 60° C beträgt.

11. Verfahren nach Anspruch 1 oder 3, worin in Schritt (b) 1,2 Äquivalente Säure zugegeben werden.

12. Verfahren nach Anspruch 2, worin in Schritt (c) 1,2 Äquivalente Säure zugegeben werden.

13. Verfahren nach Anspruch 1 oder 3, worin in Schritt (b) die Säure gewählt wird aus der Gruppe bestehend aus Schwefelsäure, Salpetersäure, Bromwasserstoffsäure, Chlorwasserstoffsäure und Phosphorsäure.

14. Verfahren nach Anspruch 2, worin in Schritt (c) die Säure gewählt wird aus der Gruppe bestehend aus Schwefelsäure, Salpetersäure, Bromwasserstoffsäure, Chlorwasserstoffsäure und Phosphorsäure.

15. Verfahren nach Anspruch 13, worin die Säure in Schritt (b) Schwefelsäure ist.

16. Verfahren nach Anspruch 14, worin die Säure in Schritt (c) Schwefelsäure ist.

17. Verfahren nach Anspruch 1, 2 oder 3, worin die organische Säure Milchsäure, 2-Keto-L-gulonsäure, Zitronensäure oder Glukonsäure umfasst.

18. Verfahren nach Anspruch 17, worin die organische Säure 2-Keto-L-gulonsäure ist.

19. Verfahren nach Anspruch 1, welches außerdem das Verestem der organischen Säure (c) zu dem korrespondierenden Ester umfasst.

20. Verfahren nach Anspruch 2, welches außerdem das Verestem der organischen Säure (d) zu dem korrespondierenden Ester umfasst.

21. Verfahren zur Gewinnung einer organischen Säure aus einer Fermentationsbrühe, umfassend:
(a) Trocknen der Fermentationsbrühe, um ein getrocknetes Produkt zu erhalten, wobei das Trocknen ohne vorherige Entfernung von Unlöslichem aus der eine organische Säure enthaltenden Fermentationsbrühe erfolgt;
(b) Zugeben des getrockneten Produkts (a) zu einem niedrigen Alkohol, gewählt aus der Gruppe bestehend aus Methanol, Ethanol, Propanol, Butanol oder Glykol, in Anwesenheit einer Säure; und
(c) Entfernen von Unlöslichem um eine organische Säure zu erhalten.

22. Verfahren nach Anspruch 21, worin in Schritt (b) die Konzentration der organischen Säure, die zu dem niedrigen Alkohol gegeben wird, 50 g/l bis 100 g/l beträgt.

23. Verfahren nach Anspruch 21, worin in Schritt (a) das Verfahren zum Trocknen das Sprühtrocknen der Fermentationsbrühe umfasst.

24. Verfahren nach Anspruch 21, worin die Reaktionstemperatur in Schritt (b) 25° bis 60° C beträgt.

25. Verfahren nach Anspruch 21, worin in Schritt (b) 1,2 Äquivalente Säure zugegeben werden.

26. Verfahren nach Anspruch 21, worin in Schritt (b) die Säure gewählt wird aus der Gruppe bestehend aus Schwefelsäure, Salpetersäure, Bromwasserstoffsäure, Chlorwasserstoffsäure und Phosphorsäure.

27. Verfahren nach Anspruch 26, worin die Säure in Schritt (b) Schwefelsäure ist.

28. Verfahren nach Anspruch 21, worin die organische Säure Milchsäure, 2-Keto-L-gulonsäure, Zitronensäure oder Glukonsäure umfasst.

29. Verfahren nach Anspruch 28, worin die organische Säure 2-Keto-L-gulonsäure ist.

30. Verfahren nach Anspruch 21, worin in Schritt (c) das Verfahren zum Abtrennen von Unlöslichem Filtration umfasst.

31. Verfahren nach Anspruch 21, welches außerdem das Verestem der organischen Säure (c) zu dem korrespondiereden Ester umfasst.

## Revendications

1. Procédé pour la récupération d'un acide organique à partir d'un bouillon de fermentation, comportant :
(a) le séchage dudit bouillon de fermentation pour obtenir un produit séché ;
(b) l'addition dudit produit séché (a) à un alcool inférieur, sélectionné dans le groupe constitué de méthanol, éthanol, propanol, butanol ou glycol, en présence d'un acide ; et
(c) l'élimination d'insolubles pour obtenir un acide organique, le procédé d'élimination d'insolubles comportant une filtration.

2. Procédé pour la récupération d'un acide organique à partir d'un bouillon de fermentation, comportant :
(a) le séchage dudit bouillon de fermentation pour obtenir un produit séché ;
(b) l'addition dudit produit séché (a) à un alcool inférieur, sélectionné dans le groupe constitué de méthanol, éthanol, propanol, butanol ou glycol, pour obtenir une suspension alcoolique ;
(c) l'addition d'un acide à ladite suspension alcoolique (b) ; et
(d) l'élimination d'insolubles pour obtenir un acide organique, le procédé d'élimination d'insolubles comportant une filtration.

3. Procédé pour la récupération d'un ester d'acide organique à partir d'un bouillon de fermentation, comportant :
(a) le séchage dudit bouillon de fermentation pour obtenir un produit séché ;
(b) l'addition dudit produit séché (a) à un alcool inférieur, sélectionné dans le groupe constitué de méthanol, éthanol, propanol, butanol ou glycol, en présence d'un acide ;
(c) l'estérification de l'acide organique libre en l'ester correspondant ; et
(d) l'élimination d'insolubles pour obtenir un ester d'acide organique, le procédé d'élimination d'insolubles comportant une filtration.

4. Procédé selon la revendication 1, 2, ou 3, comportant de plus l'élimination d'insolubles dans ledit bouillon de fermentation avant le séchage de l'étape (a).

5. Procédé selon la revendication 4, dans lequel lesdits insolubles sont éliminés par filtration.

6. Procédé selon la revendication 5, dans lequel lesdits insolubles sont éliminés par ultrafiltration.

7. Procédé selon la revendication 1, 2 ou 3, dans lequel à l'étape (b) la concentration dudit acide organique ajouté audit alcool inférieur est comprise entre 50 g/L et 100 g/L.

8. Procédé selon la revendication 1, 2 ou 3, dans lequel à l'étape (a) le procédé de séchage comporte un séchage par pulvérisation dudit bouillon de fermentation.

9. Procédé selon la revendication 1, dans lequel la température de réaction à l'étape (b) est comprise entre 25°C et 60°C.

10. Procédé selon la revendication 2 ou 3, dans lequel la température de réaction aux étapes (b) et (c) est comprise entre 25°C et 60°C.

11. Procédé selon la revendication 1 ou 3, dans lequel à l'étape (b) 1,2 équivalent d'acide est ajouté.

12. Procédé selon la revendication 2, dans lequel à l'étape (c) 1,2 équivalent d'acide est ajouté.

13. Procédé selon la revendication 1 ou 3, dans lequel à l'étape (b) ledit acide est sélectionné dans le groupe constitué d'acide sulfurique, acide nitrique, acide bromhydrique, acide chlorhydrique et acide phosphorique.

14. Procédé selon la revendication 2, dans lequel à l'étape (c) ledit acide est sélectionné dans le groupe constitué d'acide sulfurique, acide nitrique, acide bromhydrique, acide chlorhydrique et acide phosphorique.

15. Procédé selon la revendication 13, dans lequel à l'étape (b) ledit acide est l'acide sulfurique.

16. Procédé selon la revendication 14, dans lequel à l'étape (c) ledit acide est l'acide sulfurique.

17. Procédé selon la revendication 1, 2 ou 3, dans lequel ledit acide organique comporte de l'acide lactique, de l'acide 2-céto-L-gulonique, de l'acide citrique ou de l'acide gluconique.

18. Procédé selon la revendication 17, dans lequel ledit acide organique est de l'acide 2-céto-L-gulonique.

19. Procédé selon la revendication 1, comportant de plus l'estérification dudit acide organique (c) en l'ester correspondant.

20. Procédé selon la revendication 2, comportant de plus l'estérification dudit acide organique (d) en l'ester correspondant.

21. Procédé pour la récupération d'un acide organique à partir d'un bouillon de fermentation, comportant :
(a) le séchage dudit bouillon de fermentation pour obtenir un produit séché, ledit séchage ayant lieu sans élimination antérieure d'insolubles dudit bouillon de fermentation contenant un acide organique ;
(b) l'addition dudit produit séché (a) à un alcool inférieur sélectionné dans le groupe constitué de méthanol, éthanol, propanol, butanol ou glycol, en présence d'un acide ; et
(c) l'élimination d'insolubles pour obtenir un acide organique.

22. Procédé selon la revendication 21, dans lequel à l'étape (b) la concentration dudit acide organique ajouté audit alcool inférieur est comprise entre 50 g/L et 100 g/L.

23. Procédé selon la revendication 21, dans lequel à l'étape (a) le procédé de séchage comporte un séchage par pulvérisation dudit bouillon de fermentation.

24. Procédé selon la revendication 21, dans lequel la température de réaction à l'étape (b) est comprise entre 25°C et 60°C.

25. Procédé selon la revendication 21, dans lequel à l'étape (b) 1,2 équivalent d'acide est ajouté.

26. Procédé selon la revendication 21, dans lequel à l'étape (b) ledit acide est sélectionné dans le groupe constitué d'acide sulfurique, acide nitrique, acide bromhydrique, acide chlorhydrique et acide phosphorique.

27. Procédé selon la revendication 26, dans lequel à l'étape (b) ledit acide est l'acide sulfurique.

28. Procédé selon la revendication 21, dans lequel ledit acide organique comporte de l'acide lactique, de l'acide 2-céto-L-gulonique, de l'acide citrique ou de l'acide gluconique.

29. Procédé selon la revendication 28, dans lequel ledit acide organique est de l'acide 2-céto-L-gulonique.

30. Procédé selon la revendication 21, dans lequel à l'étape (c) le procédé d'élimination d'insolubles comporte une filtration.

31. Procédé selon la revendication 21, comportant de plus l'estérification dudit acide organique (c) en l'ester correspondant.
